# EUROPEAN PATENT APPLICATION

(11) **EP 2 481 417 A1**
(43) Date of publication of application: **01.08.2012**
(21) Application number: 10818733.7
(22) Date of filing: 16.09.2010
(51) Int. Cl.: A61K 38/46, A23L 1/30, A61K 31/194, A61K 33/00, A61K 33/10, A61K 33/42, A61P 1/04, A61P 1/12, A61P 1/14

(54) **LACTASE PREPARATION**

(30) Priority: 22.09.2009 JP 2009218288
(71) Applicant: Amano Enzyme Inc., Nagoya-shi, Aichi 460-8630 (JP)
(72) Inventor: GOTO Masataka, Kakamigahara-shi Gifu 509-0109 (JP); HIROSE Yoshihiko, Kakamigahara-shi Gifu 509-0109 (JP)
(74) Representative: Friede, Thomas
(86) International application number: PCT/JP2010/066015
(87) International publication number: WO 2011/037058

(57) **Abstract**

Disclosed is a lactase preparation which does not undergo the deactivation of lactase even when the preparation is ingested prior to the ingestion of a lactase-containing food or drink such as a milk product and therefore can decompose lactase stably and which can prevent the symptoms of lactase intolerance. Specifically disclosed is a lactase preparation comprising lactase and at least one component selected from trisodium citrate, potassium sodium tartrate, calcium carbonate, sodium carbonate and calcium hydrogen phosphate which acts as an antacid. The lactase preparation is characterized by being intended to be ingested prior to the ingestion of a lactase-containing food or drink to decompose lactase. In the lactase preparation, the antacid is contained at such a content per unit dosage form that the pH value in the stomach can be increased to 4 or higher after the ingestion of the preparation.

## Description

### Field of the invention

The present invention relates to a lactase preparation degrading lactose in lactose-containing food or drink such as milk products (hereinafter, also referred to as simply milk products) , and more particularly to a lactase preparation that can deliver the lactase stably to the stomach without deactivation of the lactase to degrade lactose in a milk product even when taken before ingestion of the milk product.

### Background of the invention

Lactose intolerance is caused by a lack of lactase, the digestive enzyme required to degrade lactose in the gastrointestinal system. A lactose intolerant individual produces symptoms such as indigestion, diarrhoea, and abdominal bloating upon ingestion of a milk product. It is said that there are a great number of patients with lactose intolerance in the world. In many countries including Japan and the United States, lactase (β-galactosidase) preparations are manufactured and sold, that are taken at the point of ingestion of a milk product in order to control symptoms of lactose intolerance. There has been a report that lactase can reduce or lose its activity when taken before ingestion of a milk product, for example it exhibits no effect when taken 30 minutes before drinking cow milk (see, Non-patent Literature 1). Lactase preparations are thus suggested to be taken at the point of ingestion of a milk product.

Such a suggestion is a bother for patients with lactose intolerance, because they should be careful to take lactase not before but at the point of ingestion of a milk product. In cases of ingesting only a milk product, for example drinking cow milk or eating yogurt, all they need to do is to take lactase at the point of ingestion. However, in cases where a variety of foods and drinks including milk products and others are provided, for example in a party, it is bothersome for them to take lactase when they ingest a milk product among foods and drinks, and they sometimes take lactase at the start of a party. In such a case, when they ingest a milk product among foods and drinks, lactase has reduced its activity over the time from being taken and sometimes may fail to control symptoms of lactose intolerance.
As thus, lactase must be taken with meticulous care or may fail to control symptoms of lactose intolerance due to improper taking. Therefore, there is a strong demand from patients with lactose intolerance for a lactase preparation that can control symptoms of lactose intolerance even when it is taken before ingestion of a milk product.

### References cited

### Non-patent Literature

Non-patent Literature 1: K. P. Gao, T. Mitsui, K. Fujiki, H. Ishiguro, T. Kondo, Nagoya J Med Sci 65, 21 (May, 2002)

### Summary of the invention

### Problem to be solved by the invention

Considering the circumstances, the object of the present invention is to provide a lactase preparation that can deliver lactase stably without deactivation of the lactase to degrade lactose in a milk product even when the preparation is taken before ingestion of the product and can control symptoms of lactose intolerance.

### Means for solving the problem

In order to solve the problem, the inventors have investigated in various ways and accomplished the present invention.
The present invention provides a lactase preparation that comprises lactase and at least one antacid selected from the group consisting of trisodium citrate, potassium sodium tartrate, calcium carbonate, sodium carbonate, and calcium hydrogen phosphate, and is taken before ingestion of a lactose-containing food or drink for degrading lactose. In the present invention, the point of taking before ingestion of a lactose-containing food or drink is within 30 minutes before the ingestion.

In the present invention, the lactase may be a product by a microorganism of the genus Aspergillus or Penicillium. Examples of the microorganism of the genus Aspergillus include Aspergillus oryzae and Aspergillus niger. Examples of the microorganism of the genus Penicillium include Penicillium multicolor.

In the present invention, a content of the antacid in a dose may be an amount such that pH in a stomach is increased to 4 or higher by the dose.

The present invention also relates to a method for producing the lactase preparation that increases pH in a stomach with the antacid and degrades lactose with the lactase. In the present invention, the preparation may increase pH in the stomach to 4 or higher.

The present invention also relates to use of lactase in the form of the lactase preparation for degrading lactose in the body.

The present invention also relates to use of the antacid in the form of the lactase preparation for increasing pH in the stomach. In the present invention, the preparation may increase pH in a stomach to 4 or higher.

The present invention also relates to use of the lactase preparation for preventing lactose intolerance.

### Effects of the Invention

The lactase preparation of the present invention can deliver lactase stably without deactivation and degrade lactose even when taken before ingestion of a milk product, or the lactase preparation can be taken without meticulous care. Therefore, the lactase preparation provides immeasurable benefits to patients with lactose intolerance.

According to the method of the present invention, produced is a lactase preparation that increases pH in the stomach to make lactase degrade lactose. The method of the present invention thus can produce a lactase preparation that will degrade lactose even when taken before ingestion of a milk product.

According to the use of lactase of the present invention, lactase can degrade lactose even when the lactase preparation is taken before ingestion of a milk product. Lactase thus can be used effectively for controlling symptoms of lactose intolerance.

According to the use of the antacid of the present invention, the antacid increases pH in the stomach and makes lactase degrade lactose even when the lactase preparation is taken before ingestion of a milk product. The antacid thus can be used effectively for controlling symptoms of lactose intolerance.

According to the method for preventing lactose intolerance of the present invention, the lactase preparation is taken before ingestion of a milk product and reliably controls the symptoms of lactose intolerance.

### Brief Description of the Drawings

Fig. 1 is a graph showing residual activities of lactase after standing for a given time together with pepsin solutions of pHs 1, 2, 3, 4, and 5 (an activity of lactase before adding a pepsin solution of pH 1, 2, 3, 4, or 5 is set to 100%).
Fig. 2 is a graph showing changes of residual activity of lactase in a stomach model over time in cases of using (taking) lactase at the point and 30 minutes before the use (ingestion) of caw milk, in which A. oryzae refers to Aspergillus oryzae, and P. multicolor refers to Penicillium multicolor. The same applies to Figs. 3 to 5.
Fig. 3 is a graph showing changes of pH in a stomach model over time in cases of using (taking) lactase at the point and 30 minutes before the use (ingestion) of caw milk.
Fig. 4 is a graph showing changes of amount of glucose generated in a stomach model over time in cases of using (taking) lactase at the point and 30 minutes before the use (ingestion) of caw milk.
Fig. 5 is a graph showing changes of rate of lactose degradation in a stomach model over time in cases of using (taking) lactase at the point and 30 minutes before the use (ingestion) of caw milk.
Fig. 6 is a graph showing changes of pH with various amounts and kinds of antacids in a stomach model, in which Citrate refers to trisodium citrate, Tartarate refers to potassium sodium tartrate, Magnesium Aminometasilicate refers to magnesium aluminometasilicate, and Hydrotalcite refers to synthetic hydrotalcite. The same applies to Figs. 7 to 10.
Fig. 7 is a graph showing changes of residual activity of lactase in a stomach model with various amounts and kinds of antacids over time.
Fig. 8 is a graph showing changes of residual activity of lactase in a stomach model with various amounts and kinds of antacids over time.
Fig. 9 is a graph showing changes of amount of glucose generated in a stomach model with various amounts and kinds of antacids over time.
Fig. 10 is a graph showing changes of amount of glucose generated in a stomach model with various amounts and kinds of antacids over time.

### Mode for carrying out the invention

Any source can be used for producing lactase. Examples of the source include Aspergillus oryzae, Aspergillus niger, Penicilliummulticolor, Kluyveromyces fragilis, Kluyveromyces lactis, and Bacillus circulans. Among products from these sources, those having established higher safety from Aspergillus niger and Penicillium multicolor are preferred, and a product from Aspergillus oryzae is more preferred. Such lactase may be a commercial product. An amount of lactase used can be appropriately determined in consideration of age, sex, weight, and a degree of symptoms, and the like, of a patient with lactose intolerance.

The lactase preparation of the present invention comprises at least one antacid selected from the group consisting of trisodium citrate, potassium sodium tartrate, calcium carbonate, sodium carbonate, and calcium hydrogen phosphate. The antacid can increase pH in a stomach to prevent deactivation of lactase. A commercially available antacid may also be used. A content of the antacid in a dose is preferably an amount such that pH in the stomach is increased to 4 or higher. Excessively increased pH in the stomach, however, may lead gastric acid secretion as a rebound reaction. The content is thus preferably not more than the amount that increases pH in the stomach to 7.

The lactase preparation of the present invention can be of any form if it can be taken orally. Examples of the form include powder, fine granule, granule, pill, tablet, capsule, soft capsule, troche, and syrup. The lactase preparation of the present invention can further comprise any type of additive if the additive does not have effects on pH in a stomach. Examples of the additive include excipient, binder, lubricant, disintegrant, and preservative. Specific examples of the excipient include potato starch, corn starch, sucrose, mannitol, sorbitol, and talc. Specific examples of the binder include starch, α-starch, dextrin, hydroxypropyl starch, methyl cellulose, and carboxy methyl cellulose. Specific examples of the lubricant include aluminium stearate, magnesium stearate, calcium stearate, and polyethylene glycol. Specific examples of the disintegrant include carboxy methyl starch sodium, carmellose calcium, carmellose, corn starch, and lactose. Specific examples of the preservative include parahydroxybenzoates, chlorobutanol, and benzyl alcohol.

The lactase preparation of the present invention can deliver lactase without deactivation of lactase to degrade lactose even when the preparation is taken before ingestion of a lactose-containing food or drink. Examples of the lactose-containing food or drink include milk products such as cow milk, yogurt, cheese, butter, cream, and powdered milk.

The subject to which the lactase preparation of the present invention can be applied is preferably human, but may also be other mammal such as dog, cat, cow, pig, chicken, monkey, sheep, and goat.

The lactase preparation of the present invention is taken within 30 minutes before ingestion of a lactose-containing food or drink.

### Examples

Next, the present invention will be described with reference to Examples, but should not be limited thereto.

### [Example 1] (Study of lactase about pH stability with a pepsin solution at various pH values)

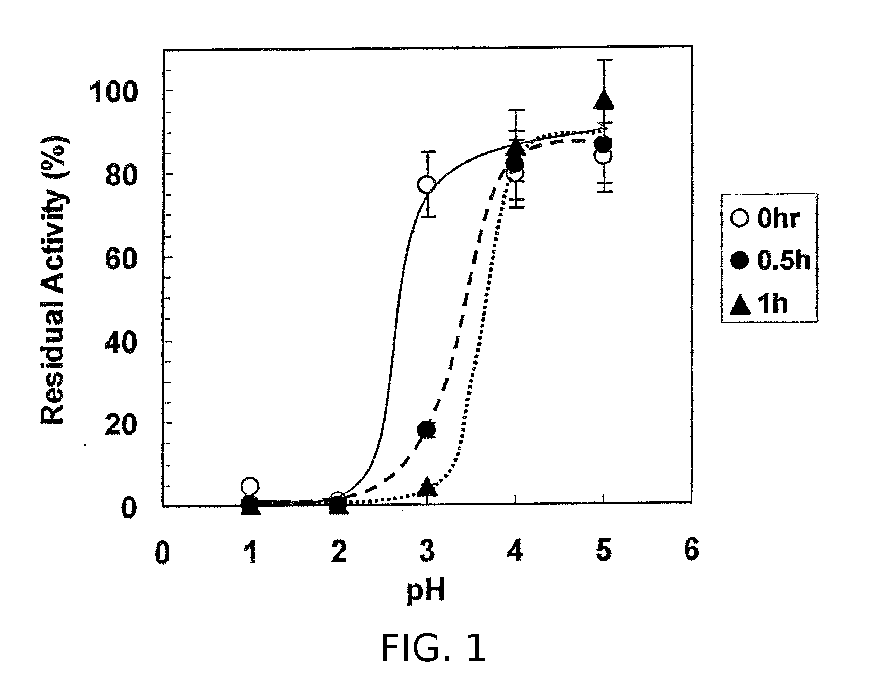

Stability of lactase at various pH values was examined by adding pepsin solutions at pHs 1, 2, 3, 4, and 5 to lactase, and allowing each mixture to stand for a predetermined period.
To each 0.4 mL of lactase derived from Aspergillus oryzae at 3000 ALU/mL (Amano Enzyme Inc., lactase DS) were added 0.2 mL of 0.2wt% pepsin (Sigma) and each 5.4 mL of 0.1M acetic acid-NaCl solutions at pHs 1, 2, 3, 4, and 5 to prepare pepsin solutions at pHs 1, 2, 3, 4, and 5. These solutions were allowed to stand for 0, 0.5, and 1 hour at 37°C, and diluted 10 times with 0.1M sodium acetate buffer (pH 4.5). Diluted solutions were used to measure a residual activity of lactase with a pepsin solution at various pH values by FCC IV. FCC IV is an assay based on a 15 minutes hydrolysis of an o-nitrophenyl-β-D-galactopyranoside substrate in an acetate buffer at 37°C and pH 4.5, and measures an absorbance at 420 nm with a spectrophotometer (FOOD CHEMICALS CODEX 4th EDITION, Effective July 1, 1996, COMMITTEE ON FOOD CHEMICALS CODEX, Food and Nutrition Board Institute of Medicine National Academy of Sciences, NATIONAL ACADEMY PRESS, Washington, D.C. 1996). Results are shown in Fig. 1. In the measurement, an amount of enzyme at which the reaction of lactase with o-nitrophenyl-β-D-galactopyranoside as the substrate for 15 minutes at 37°C and pH 4.5 releases 1 µmol/min of o-nitrophenol was set to one unit (1 ALU).

As shown in Fig. 1, lactase exhibited a residual activity of about 60% at pH 3.5 after 0.5 hours standing and about 80% or higher at pH 4.0 or higher regardless of a standing time. At pH 3, lactase tended to be more deactivated with the longer standing time, and exhibited a residual activity of about 80% after 0 hours standing, about 20% after 0.5 hours, and about 5% after 1 hour. At pH 2 or lower, lactase was immediately deactivated regardless of the length of standing time. These results suggest that when lactase is taken on an empty stomach, since pH in the empty stomach is generally not more than 2, it is deactivated, while lactase can hold its activity sufficiently even in a stomach if pH in the stomach is 4 or higher.

### [Example 2] (Study of lactase about lactose degradation in a stomach model)

A stomach model was made and used to study lactose degradation by lactase in cases where lactase was used (taken) at the point of use (ingestion) of cow milk and 30 minutes before the use (ingestion) of cow milk.

### (Example 2-1) Case where lactase was used (taken) at the point of use (ingestion) of cow milk

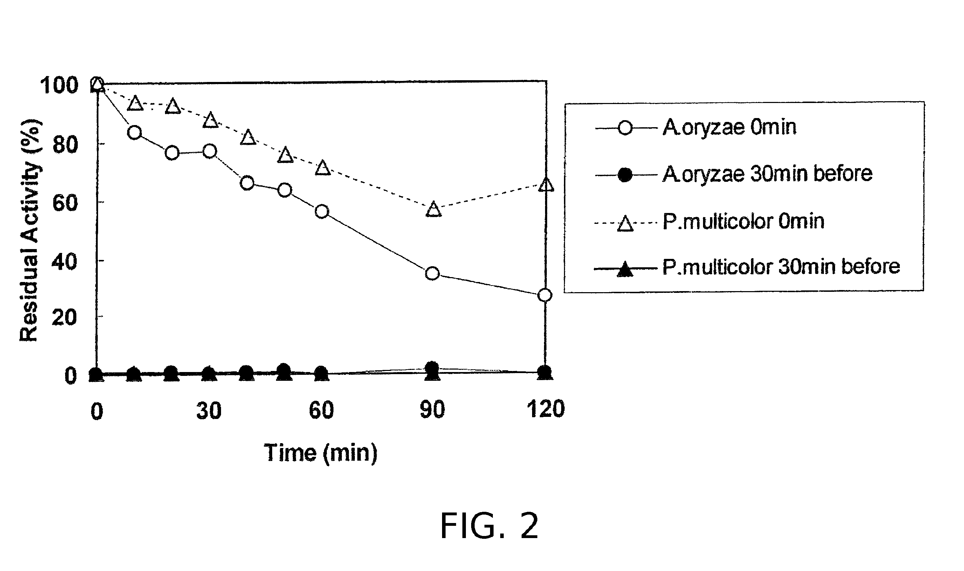

100 mL of commercial milk, 5 mL of 3.0wt% mucin (Nacalai Tesque, Inc.), and 10 mL of 2.25M NaCl-15mM CaCl₂ were mixed. To this was added 6.5 mL of 0.1N HCl so as to be pH 6. To the mixture was further added 13.5 mL of water to give 150 mL of solution (final concentrations: 0.1% mucin, 150mM NaCl, and 1mM CaCl₂) . To the solution were added 5 mL of 0.2wt% pepsin (Sigma) (10 mg) and 10 mL of lactase (3000 ALU). The mixture was incubated at 37°C to start a reaction in a stomach model. Lactose degradation was examined with addition of 0.5N HCl every one minute in amounts of 0.5 mL from minute 0 to 5, 0.15 mL from minute 6 to 25, 0.3 mL from minute 26 to 35, 0.15 mL from minute 36 to 55, and 0.3 mL from minute 55 to 65 to mimic a pH change in a stomach. Samples were taken every 10 minutes from minute 0 to 60 and at minute 90 and minute 120.
Samples of the solution were measured about pH, a residual activity of lactase and an amount of glucose generated. The residual activity of lactase was measured by FCC IV as described. Measurement of the amount of glucose generated and calculation of a rate of lactose degradation were as described below. In this example, lactase derived from Aspergillus oryzae (Amano Enzyme Inc. , lactase DS) and lactase derived from Penicillium multicolor (Shionogi & Co., Ltd., Millact) were used. Results are shown in Figs. 2 to 5.

An amount of glucose was measured as follows. To 9 µL of sample was added 350 µL of reagent 1 and incubated for 5 minutes at 37°C. To the sample was then added 75 µL of reagent 2 and reacted for 4 minutes at 37°C. NADH generated in the sample was measured at 340 nm to quantify an amount of glucose.
The reagent 1 was a buffer solution of pH 7.0 prepared such that it contained mutarotase (Amano Enzyme Inc., MUT"Amano"2) at a final concentration of 2.5 u/mL, sodium dehydroacetate at a final concentration of 0.02wt%, Triton X-100 at a final concentration of 0.15wt%, EDTA at a final concentration of 1mM, β-NAD at a final concentration of 1mM, and BSE at a final concentration of 100mM. The reagent 2 was a buffer solution of pH 7.0 prepared such that it contained glucose dehydrogenase (Amano Enzyme Inc., GLUCDH"Amano"2) at a final concentration of 80 u/mL, EDTA at a final concentration of 1mM, Triton X-100 at a final concentration of 0.15wt%, sodium dehydroacetate at a final concentration of 0.02wt%, and BSE at a final concentration of 100mM.
A rate of lactose degradation was calculated as follows. To cow milk was added sulfuric acid so as to be 1N sulfuric acid. The milk was heated to 100°C for 18 hours to hydrolyze. The milk was then neutralized with calcium carbonate. An amount of glucose generated in the milk was quantified as described above. A mole number of the glucose was considered as a mole number of lactose in the milk.
The mole number of lactose in the milk was used as 100 to calculate a ratio of a mole number of glucose generated by lactase.

### (Example 2-2) Case where lactase was used (taken) 30 minutes before the use (ingestion) of cow milk

To a mixture of 0.55 mL of 3.0wt% mucin (Nacalai Tesque, Inc.), 0.11 mL of 2.25M NaCl-15mM CaCl₂, 0.55 mL of 0.2% pepsin (Sigma), and 7.15 mL of 0.1N HCl was added 11 mL of lactase (3000 ALU) and incubated for 30 minutes at 37°C (pH 1.36). 17.6 mL of sample was taken from the incubated solution. To the sample were added 4.5 mL of 3.0wt% mucin (Nacalai Tesque, Inc.), 9.9 mL of 2.25M NaCl-15mM CaCl₂, 4.5 mL of 0.2wt% pepsin (Sigma), 13.5 mL of water, and 100 mL of commercial milk to start a reaction in a stomach model. The following operations including addition of HCl, sampling, measurement of a residual activity of lactase, and measurement of an amount of glucose were similarly performed as in Example 2-1. Results are shown in Figs. 2 to 5.

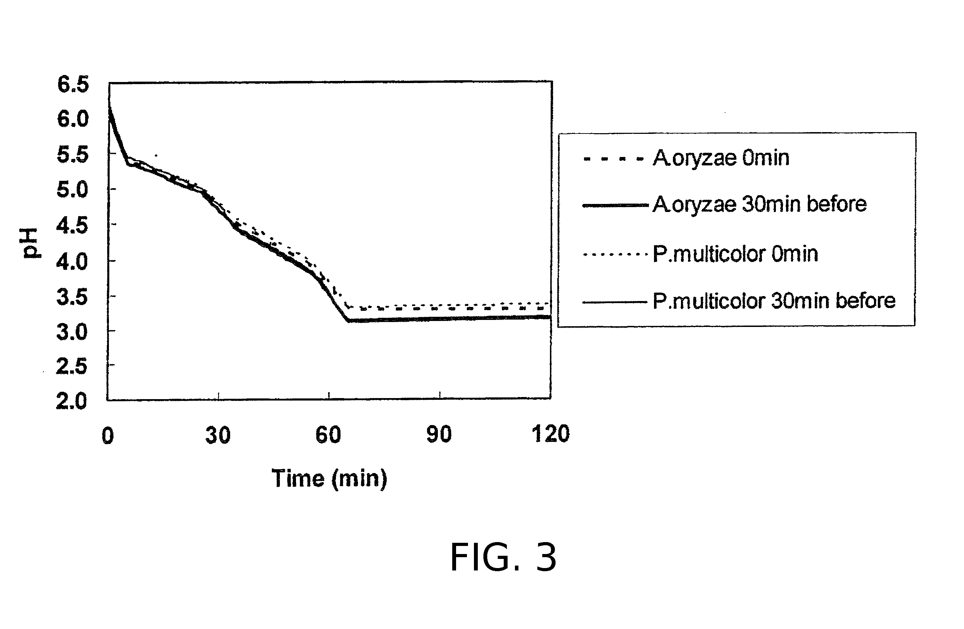

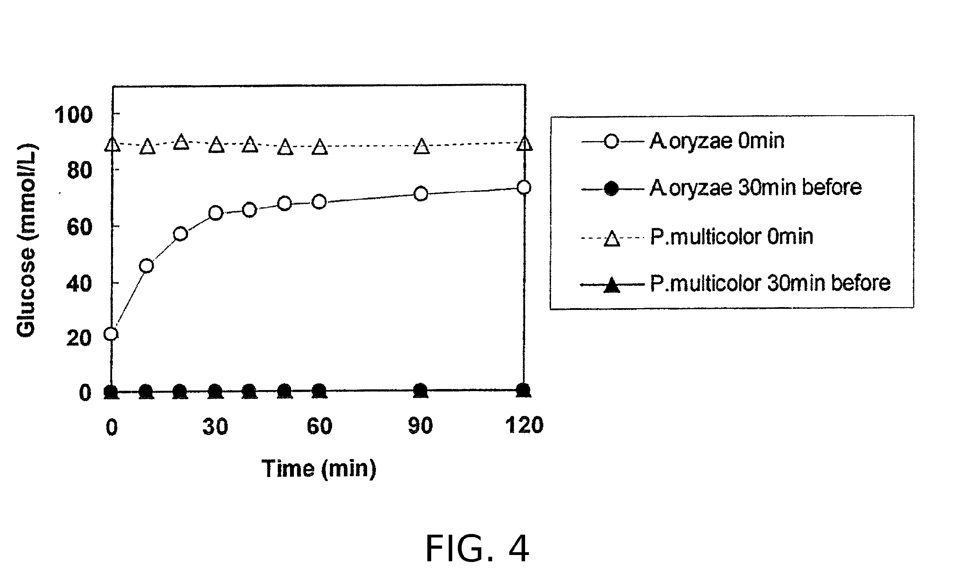

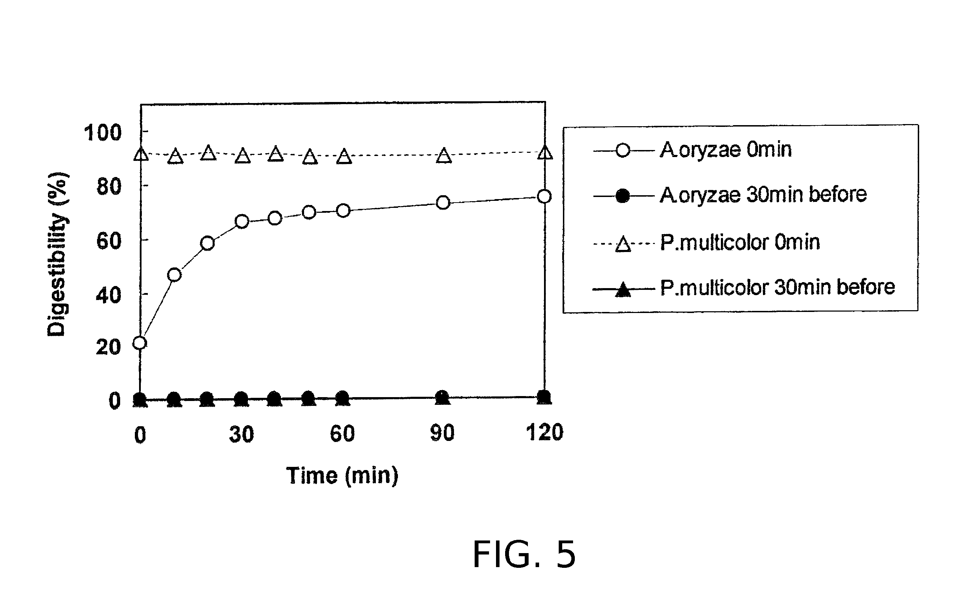

In the case of using (taking) lactase at the point of use (ingestion) of cow milk, a residual activity of lactase decreased over time. At minute 120, the lactase derived from Aspergillus oryzae exhibited a residual activity of about 35% and the lactase derived from Penicillium multicolor exhibited about 65% (see, Fig. 2). In contrast, in the case of using (taking) lactase 30 minutes before the use (ingest) of cow milk, both lactases were deactivated immediately (see, Fig. 2).
Changes of pH in a stomach model in the cases of using (taking) lactase at the point and 30 minutes before the use (ingestion) of cow milk were similar over time to each other (see, Fig. 3).
In the case of using (taking) lactase at the point of use (ingestion) of cow milk, the lactase derived from Penicillium multicolor exhibited about 90% of glucose generation and about 90% of lactose degradation immediately after the lactase was used (taken) (see Figs. 4 and 5). The lactase derived from Aspergillus oryzae exhibited increasing glucose generation and lactose degradation from immediately after the lactase was used (taken) and plateaus at about 70% reached after 30 minutes (see Figs. 4 and 5).
In the case of using (taking) lactase 30 minutes before the use (ingest) of cow milk, glucose generation and lactose degradation were not confirmed due to immediate deactivation of lactase (see Figs. 4 and 5).
These results suggest that deactivation of lactase was attributed to pH stability of the lactase.

### [Example 3] (Study of effect of various amounts and kinds of antacid on pH in a stomach model)

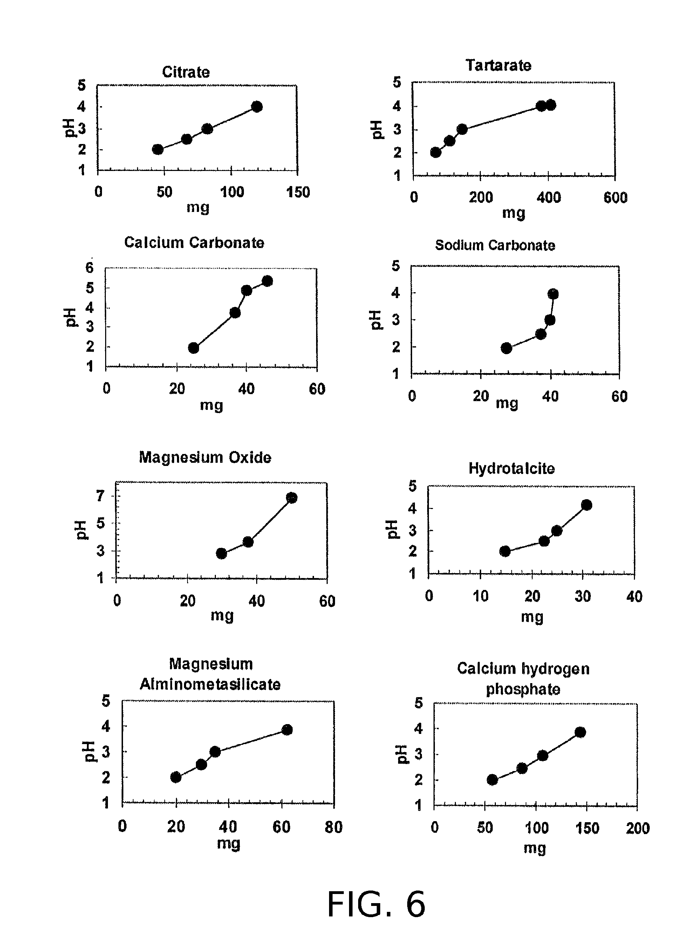

Various antacids were used in various amounts to study effects on pH in a stomach model considering with the findings in Example 1.
To a mixture of 0.55 mL of 3.0wt% mucin (Nacalai Tesque, Inc.), 0.11 mL of 2.25M NaCl-15mM CaCl₂, 0. 55 mL of 0.2% pepsin (Sigma), and 7.15 mL of 0.1N HCl was added 11 mL of lactase derived from Aspergillus oryzae (Amano Enzyme Inc., lactase DS) (3000 ALU). Various antacids selected from trisodium citrate (Katayama Chemical Industries Co., Ltd.), potassium sodium tartrate (Wako Pure Chemical Industries, Ltd.), calcium carbonate (Wako Pure Chemical Industries, Ltd.), sodium carbonate (Wako Pure Chemical Industries, Ltd.), magnesium oxide (Wako Pure Chemical Industries, Ltd.), synthetic hydrotalcite (Tomita Pharmaceutical Co., Ltd. (unofficial drug not listed in Japanese pharmacopoeia)), magnesium aluminometasilicate (Fuso Pharmaceutical Industries, Ltd.), and calcium hydrogen phosphate (Wako Pure Chemical Industries, Ltd.) were individually added to the mixture at various amounts, allowed to stand for 30 minutes at 37°C, and measured about pH. Results are shown in Fig. 6.

As shown in Fig. 6, pH in a stomach model increased with increasing amounts of respective antacids. Added amounts of antacids to increase pH to 4 were 120 mg for trisodium citrate, 413 mg for potassium sodium tartrate, 40 mg for calcium carbonate, 42 mg for sodium carbonate, 37 mg for magnesium oxide, 31 mg for synthetic hydrotalcite, 62 mg for magnesium aluminometasilicate, and 150 mg for calcium hydrogen phosphate. A pH in a stomach model can be considered as proportionally depending on a mole amount of hydrochloric acid. In this Example, 7.15 mL of 0.1N HCl was used. In a modified Fucks method that evaluates antacid effects by adding an antacid in an amount corresponding to one dosage to 50 ml of 0.1N HCl (Yamagata et al., Kiso to Rinsho (The clinical report, basic and clinical study), Vol. 24, No. 10, 1023-1028 (1990)), these antacids will increase pH to 4 in 6.99 times the respective amounts used above.

### [Example 4] (Study of lactase about lactose degradation in the presence of various antacids in a stomach model)

For the case of using (taking) lactase 30 minutes before the use (ingestion) of cow milk, studied was lactose degradation by lactase in the presence of an antacid in a stomach model.

To a mixture of 0.55 mL of 3.0wt% mucin (Nacalai Tesque, Inc.), 0. 11 mL of 2.25M NaCl-15mM CaCl₂, 0.55 mL of 0.2% pepsin (Sigma), and 7.15 mL of 0.1N HCl were added 11 mL of lactase derived from Aspergillus oryzae (Amano Enzyme Inc., lactase DS) (3000ALU) and 120 mg of trisodium citrate (Katayama Chemical Industries Co., Ltd.), of which amount was shown to increase pH in a stomach model to 4 according to Example 3, and incubated for 30 minutes at 37°C. 17.6 mL of sample was taken from the incubated solution. To the sample were added 4.5 mL of 3.0wt% mucin (Nacalai Tesque, Inc.), 9. 9 mL of 2.25M NaCl-15mM CaCl₂, 4.5 mL of 0.2wt% pepsin (Sigma), 13.5 mL of water, and 100 mL of commercial milk to start a reaction in a stomach model. The following operations including addition of HCl, sampling, measurement of a residual activity of lactase, and measurement of an amount of glucose were similarly performed as in Example 2-1.

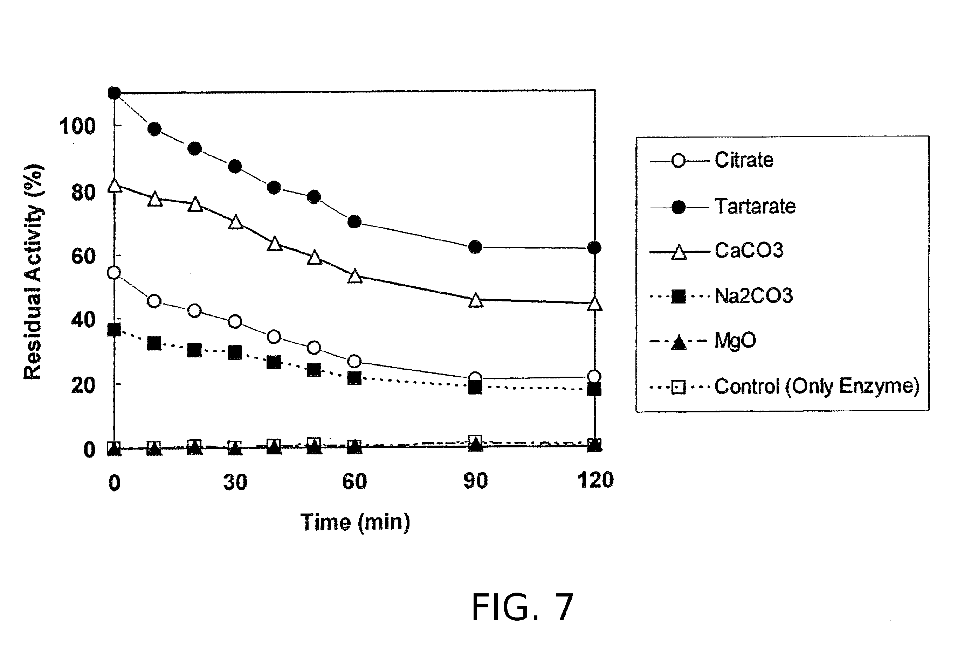

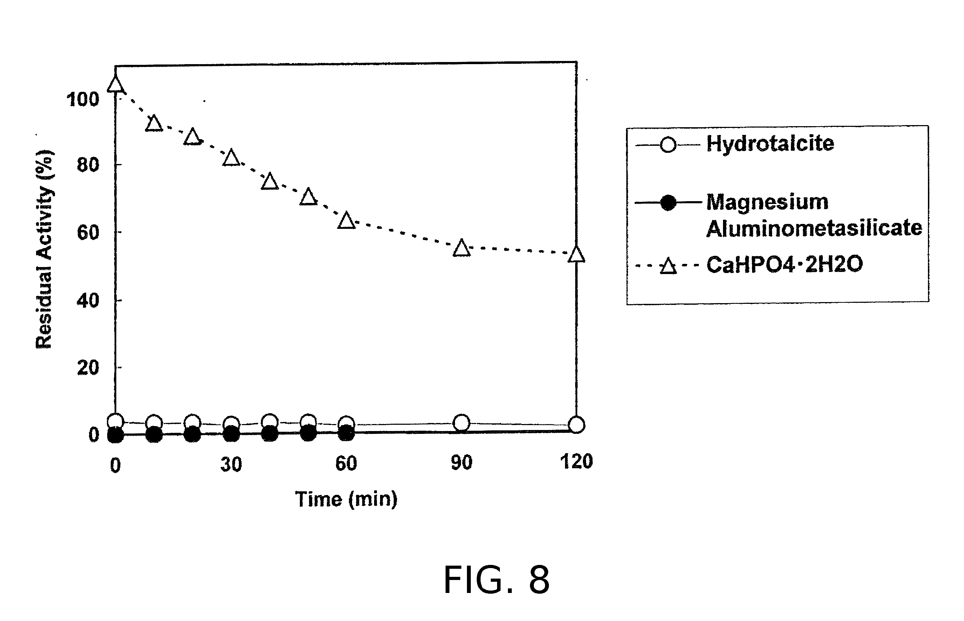

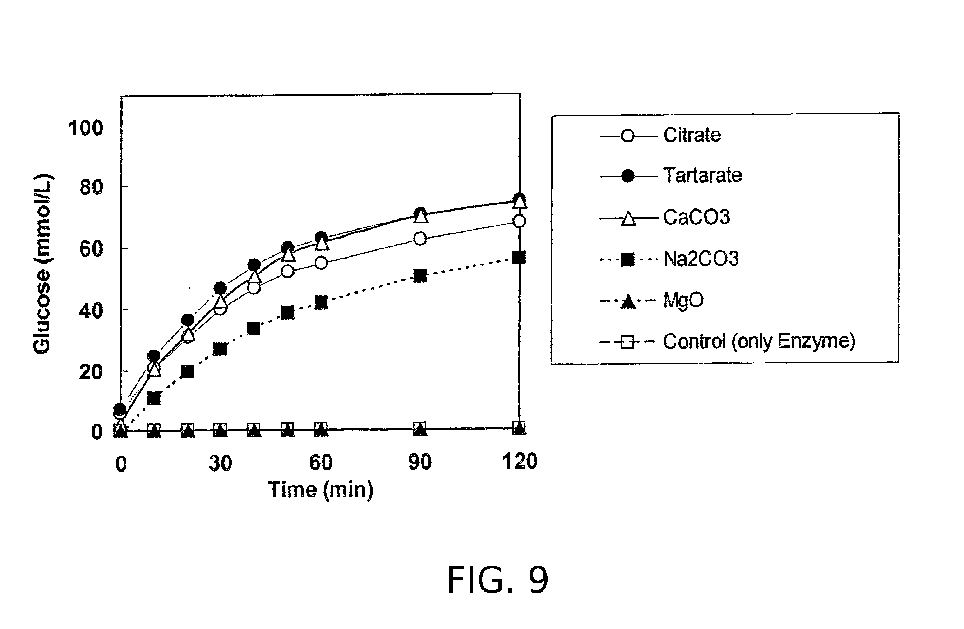

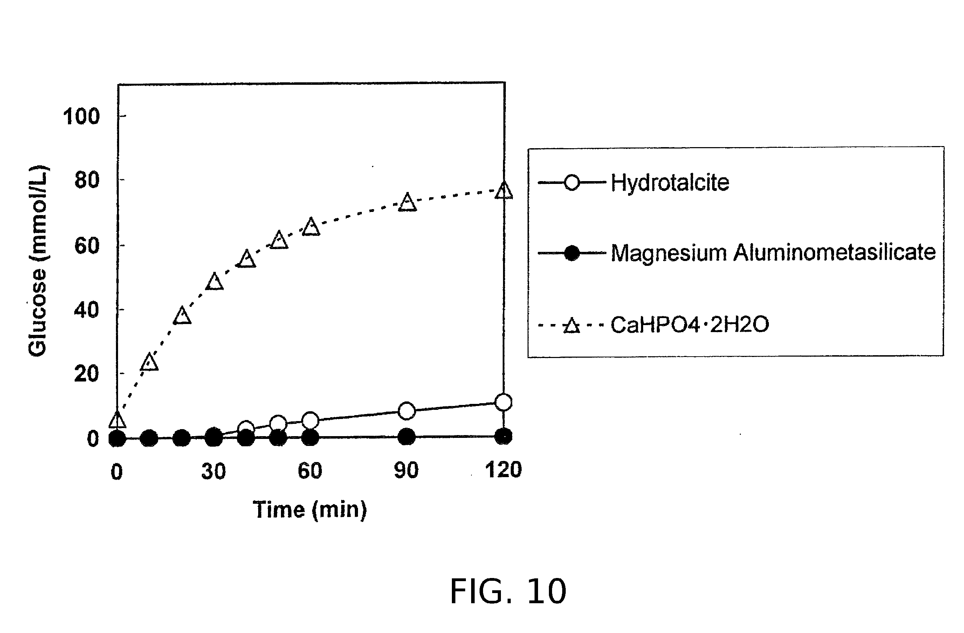

Tests with various antacids were similarly performed as above, except that respective antacids were used instead of 120 mg of trisodium citrate. Respective amounts of antacids were as follows, which were shown to increase pH in a stomach model to 4 according to Example 3 (see, Fig. 6): (1) 413 mg of potassium sodium tartrate (Wako Pure Chemical Industries, Ltd.); (2) 40 mg of calcium carbonate (Wako Pure Chemical Industries, Ltd.); (3) 42 mg of sodium carbonate (Wako Pure Chemical Industries, Ltd.) ; (4) 37 mg of magnesium oxide (Wako Pure Chemical Industries, Ltd.); (5) 31 mg of synthetic hydrotalcite (Tomita Pharmaceutical Co., Ltd. (unofficial drug not listed in Japanese pharmacopoeia)); (6) 62 mg of magnesium aluminometasilicate (Fuso Pharmaceutical Industries, Ltd.); and (7) 150 mg of calcium hydrogen phosphate (Wako Pure Chemical Industries, Ltd.).
Results are shown in Figs. 7 to 10.

In cases of adding potassium sodium tartrate, calcium carbonate, or calcium hydrogen phosphate, the lactase exhibited a residual activity equal to or higher than that of the lactase derived from Aspergillus oryzae in the case of using (taking) lactase at the point of the use (ingestion) of cow milk in Example 2-1 (see, Figs. 2, 7, and 8) . Amounts of glucose generated were slightly lower than that generated by the lactase derived from Aspergillus oryzae in the case of using (taking) lactase at the point of the use (ingestion) of cow milk in Example 2-1 (see, Figs. 4, 9, and 10). In cases of adding trisodium citrate or sodium carbonate, a residual activity of the lactase and an amount of glucose generated were slightly lower than that of the lactase derived from Aspergillus oryzae in the case of using (taking) lactase at the point of the use (ingestion) of cow milk in Example 2-1 (see, Figs. 2, 4, 7, and 9).
These results showed that lactase could degrade lactose if it was taken together with at least one antacid selected from the group consisting of trisodium citrate, potassium sodium tartrate, calcium carbonate, sodium carbonate and calcium hydrogen phosphate 30 minutes before ingestion of a milk product.
In contrast, magnesium oxide, synthetic hydrotalcite, and magnesium aluminometasilicate showed that these could not prevent deactivation of lactase when taken together with lactase 30 minutes before ingestion of a milk product (see, Figs. 7 to 10).

### Industrial applicability

The lactase preparation of the present invention is effective for lactose intolerance even when it is taken before ingestion of a milk product, and thus is useful as a medicine in the field of medicine or as a supplement in the field of food.

## Claims

1. A lactase preparation comprising lactase and at least one antacid selected from the group consisting of trisodium citrate, potassium sodium tartrate, calcium carbonate, sodium carbonate and calcium hydrogen phosphate, which is taken before ingestion of a lactose-containing food or drink for degrading lactose.

2. The lactase preparation according to claim 1,
wherein the lactase is a product by a microorganism selected from the genera Aspergillus and Penicillium.

3. The lactase preparation according to claim 2,
wherein a microorganism of the genus Aspergillus is selected from Aspergillus oryzae and Aspergillus niger, and a microorganism of the genus Penicillium is Penicillium multicolor.

4. The lactase preparation according to any one of claims 1 to 3, wherein a content of the antacid in a dose is an amount that increases pH in a stomach to 4 or higher.

5. A method for producing the lactase preparation according to any one of claims 1 to 4, wherein the lactase preparation increases pH in a stomach with the antacid and degrades lactose with the lactase.

6. The method for producing the lactase preparation according to claim 5, wherein pH in the stomach is increased to 4 or higher.

7. A use of lactase in the form of the lactase preparation according to any one of claims 1 to 4 for degrading lactose in the body.

8. A use of the antacid in the form of the lactase preparation according to any one of claims 1 to 4 for increasing pH in the stomach.

9. The use of the antacid according to claim 8, wherein pH in the stomach is increased to 4 or higher.

10. A method for preventing lactose intolerance, comprising the step of taking the lactase preparation according to any one of claims 1 to 4.
